# EUROPÄISCHE PATENTSCHRIFT

(11) **EP 2 141 620 B1**
(45) Veröffentlichungstag und Bekanntmachung des Hinweises auf die Patenterteilung: **31.10.2018**
(21) Anmeldenummer: 08405166.3
(22) Anmeldetag: 01.07.2008
(51) Int. Cl.: G06F 19/00

(54) **Insulinpumpe und Verfahren zur Steuerung einer Benutzerschnittstelle einer Insulinpumpe**
Insulin pump and method for controlling a user interface of an insulin pump
Pompe à insuline et procédé de commande d'une interface utilisateur d'une pompe à insuline

(43) Veröffentlichungstag der Anmeldung: 06.01.2010
(73) Patentinhaber: Roche Diabetes Care GmbH, 68305 Mannheim (DE); F. Hoffmann-La Roche AG, 4070 Basel (CH)
(72) Erfinder: Eberhart, Andreas, 6300 Zug (CH); Both, Marcel, 3088 Kirchlindach (CH); Krieftewirth, Michael, 3423 Ersigen (CH)
(74) Vertreter: Rentsch Partner AG

(56) Entgegenhaltungen:
- US-A- 4 731 051
- US-A1- 2004 100 505
- US-A1- 2007 118 405
- US-A1- 2007 179 434
- US-A1- 2008 033 402
- RONALD D SNODGRASS: "Smart Pump Technology", BIOMEDICAL INSTRUMENTATION & TECHNOLOGY, ARLINGTON, VA, vol. 39, no. 6, 1 November 2005 (2005-11-01), pages 444-446, XP009174815, ISSN: 0899-8205

## Beschreibung

### Technisches Gebiet

Die Erfindung betrifft eine Insulinpumpe mit einer Pumpeinrichtung, einer Steuerung für die Pumpeinrichtung, einem Speicher sowie mindestens einem Bedienungselement für den Benutzer der Insulinpumpe zur Bedienung der Insulinpumpe. Weiter betrifft die Erfindung ein Verfahren zur Steuerung einer Benutzerschnittstelle einer solchen Insulinpumpe.

### Stand der Technik

Es ist seit einiger Zeit bekannt, in der Insulintherapie (z. B. für Typ-1-Diabetes) Insulinpumpen einzusetzen. Die Insulinpumpentherapie ermöglicht die kontinuierliche Abgabe einer (veränderlichen) Basalrate von Insulin sowie von einzelnen Boli, die im Zusammenhang mit Mahlzeiten und für die Korrektur von zu hohen Glukosewerten im Blut des Patienten eingesetzt werden. Durch die kontinuierliche Abgabe der Basalrate gemäss einem benutzerspezifischen Basalratenprofil wird ein gleichmässigerer Blutzuckerspiegel erreicht und somit der Organismus des Patienten entlastet.

Entsprechend sind Infusionssysteme bekannt, die aus einer Infusionspumpe für Insulin (Insulinpumpe) und gegebenenfalls aus einem als Fernbedienung ausgebildeten Blutzuckermessgerät bestehen. In der Regel soll die Insulinpumpe möglichst kompakt ausgebildet sein, damit sie nicht aufträgt und für den Benutzer bequem zu tragen ist. Die Pumpe umfasst somit ein kleines Pumpengehäuse, in welchem eine Batterie, ein Motor mit Getriebe, Steuerungs- und Kommunikationselektronik und eine fest mit dem Infusionsschlauch verbundene Ampulle aufgenommen sind. An der Aussenseite weist die Pumpe Bedientasten sowie meist ein Display auf. Dieses Display und auch die Grösse und Anzahl der Bedientasten sind bedingt durch die anzustrebende Bauform limitiert.

Auf der Pumpe selbst lässt sich somit nur eine begrenzte Informationsmenge darstellen. Auf üblichen Displays können entweder gemessene Blutzuckerwerte und Handlungsempfehlungen (Bolus, Essen) oder das Menü der Insulinpumpe angezeigt werden. Heutige Insulinpumpensysteme sind mit einem starren Bedienkonzept ausgelegt. Während an der Insulinpumpe selbst einige Grundfunktionen bedienbar sind (z. B. das Ausschütten von Boli) sind weitere Funktionen nur über die Fernbedienung steuerbar, d. h. die vielfältigen Bedienfunktionen für die Insulinpumpe sind hauptsächlich auf die Fernbedienung ausgelagert.

Auch bei Insulinpumpen, die über keine Fernbedienung aufweisen und somit über Bedienelemente an der Pumpe gesteuert werden, hat die geforderte Miniaturisierung eine umständliche Steuerung zur Folge.

Heutige Infusionssysteme für Insulin lassen sich somit nur mit Hilfe einer Fernbedienung vollständig bzw. komfortabel bedienen. Ist diese nicht zur Hand oder sind die Bedienelemente sämtlich an der Pumpe ausgeführt, muss der Benutzer auf einige Funktionen verzichten und/oder eine erschwerte Bedienung in Kauf nehmen. Weil einige Funktionen aus Sicherheitsgründen immer auf der Pumpe realisiert werden sollen, können im Rahmen der bekannten Benutzerschnittstellen die entsprechende Anzeige und Bedienungselemente nicht weiter verkleinert werden, was der Miniaturisierung der Insulinpumpen Grenzen setzt.

Die US 2007/0179434 (Weinert et al.) zeigt ein Gerät, um eine Arzneimittelabgabe wie die Abgabe von Insulin zu bestimmen. Das Gerät umfasst eine Eingabeeinrichtung zur Eingabe von mindestens einer ersten und einer zweiten Parameterkomponente, einen Datenspeicher und einen Prozessor. Im Datenspeicher können Abbildungs-Korrelationswerte der Parameterkomponenten zu Arzneimittelabgabeinformationen gespeichert sein. Das Gerät umfasst eine Anzeigeeinheit zur Anzeige einer grafischen Benutzeroberfläche. Das Gerät kann als Infusionspumpe oder Blutglukosemessgerät ausgeführt sein und mit implantierten Sensoren verbunden sein, welche physiologische Informationen des Benutzers zur Verfügung stellen. Alle Benutzereingaben und Bolus Informationen werden mit einem Zeitstempel versehen und in einer Datenbank abgespeichert. Eine Abbildung zur Bestimmung einer Bolus Empfehlung kann 21 Abbildungen umfassen, welche die Benutzereingabe auf unterschiedliche Bolus Informationen in Abhängigkeit der Mahlzeit oder der Zeit abbilden (wie z. B. Frühstück, Mittagessen, Nachtessen auch in Abhängigkeit des Wochentags), wobei die Mahlzeit vom System automatisch bestimmt werden kann. Der empfohlene Bolus wird angezeigt und der Benutzer kann diesen akzeptieren oder verwerfen, z. B. mittels grafischer Anzeigen. Nach einem Verwerfen erhält der Benutzer die Möglichkeit, den empfohlenen Bolus zu verändern. Das System wird feineingestellt durch die Interaktion mit dem Patienten und zu diesem Zweck kann das System in einen Lernmodus umgeschaltet werden, so dass die Aktivitäten des Benutzers ständig aufgezeichnet werden US 2004/100505 (Cazier) handelt von Prioritisierung von Menüinformationen, aber außerhalb des Kontextes von Insulinpumpen und ohne die Aufzeichnung eines benutzerspezifischen Präferenzprofils.

### Darstellung der Erfindung

Aufgabe der Erfindung ist es, eine dem eingangs genannten technischen Gebiet zugehörende Insulinpumpe und ein Verfahren zur Steuerung einer Benutzerschnittstelle einer solchen Insulinpumpe zu schaffen, welche einen hohen Funktionsumfang und eine einfache Bedienung auch auf miniaturisierten Pumpen ermöglichen.

Die Lösung der Aufgabe ist durch die Merkmale der Ansprüche 1 und 13 definiert. Gemäss der Erfindung werden Auswahlmöglichkeiten für den Benutzer der Insulinpumpe in Abhängigkeit eines benutzerspezifischen, im Speicher der Insulinpumpe abgelegten Präferenzprofils und einer aktuellen Zeit zur Auswahl durch die Bedienungselemente der Insulinpumpe angeboten. Wie nachfolgend erläutert, umfasst die Insulinpumpe eine Anzeigeeinrichtung und die Auswahlmöglichkeiten werden auf der Anzeigeeinrichtung als Menüauswahl mit Menüpunkten angeboten. Die Steuerung ist eingerichtet und programmiert, dass die Reihenfolge der Menüpunkte der Menüauswahl gemäss dem aktuellen Präferenzprofil ausgegeben wird.

Somit erfolgt das Anbieten der Auswahlmöglichkeiten im Rahmen der erfindungsgemässen Benutzerschnittstelle benutzerspezifisch. Das Präferenzprofil gibt in Abhängigkeit der Zeit an, welche Funktionen vom jeweiligen Benutzer bevorzugt ausgewählt werden bzw. welche Funktionen zur gegebenen Zeit dem Benutzer zur Auswahl anzubieten sind (beispielsweise weil diese im Rahmen einer optimalen Therapie aufgerufen werden sollten).

Der Benutzer kann die für ihn wichtigen Funktionen somit schnell und einfach erreichen, auch wenn grundsätzlich eine grosse Zahl von Auswahlmöglichkeiten angeboten wird. Der Umfang der darzustellenden Information (z. B. die Anzahl der Auswahlmöglichkeiten) kann gleichzeitig reduziert werden, wodurch eine bessere Übersichtlichkeit geschaffen und der Platzbedarf verringert wird. Die Pumpe kann somit sehr klein ausgebildet werden, kann aber - beim Vorliegen einer Fernbedienung - trotzdem über Minimalfunktionen bedient werden, die schnell erreichbar sind und in maximaler Grösse auf der Anzeigeeinrichtung dargestellt werden - bzw. bei Systemen ohne Fernbedienung wird trotz des beschränkten Platzangebots für die Bedienelemente und die Anzeigeeinrichtung eine komfortable Bedienung aller Funktionen ermöglicht.

Im einfachsten Fall werden die Auswahlmöglichkeiten auf eine bestimmte Unterauswahl eingeschränkt, die sich aus dem Präferenzprofil und der aktuellen Zeit ergibt. Dies kann so weit gehen, dass z. B. nur eine einzige Funktion durch Druck auf eine vorbestimmte Taste auslösbar ist, wobei die Zuordnung der Funktion auf der Basis des Präferenzprofils erfolgt und sich dadurch zeitlich ändern kann.

Umfasst die Insulinpumpe eine Anzeigeeinrichtung, können aber auch Auswahlmöglichkeiten für den Benutzer auf der Anzeigeeinrichtung in Abhängigkeit des benutzerspezifischen Präferenzprofils und der aktuellen Zeit dargestellt und zur Auswahl durch das mindestens eine Bedienungselement angeboten werden. Die Anzeige auf der Anzeigeeinrichtung und die auswählbaren Menüpunkte werden somit in Abhängigkeit der aktuellen Zeit und des im Speicher abgelegten Präferenzprofils generiert. Beispielsweise kann zum Zeitpunkt der regelmässigen Bolusabgabe die Anzeige auf "Bolusmenge" voreingestellt sein; zum Zeitpunkt regelmässiger Erniedrigung der Basalrate (z. B. in einer Aktivitätsphase des Benutzers) sind das temporäre Basalratenmenü und die entsprechende Darstellung aktiviert; und in der Ruhephase des Benutzers bzw. zum Zeitpunkt eines Alarms ist die Uhrzeit bzw. Weckzeit aktiv. Entsprechend werden in Abhängigkeit der genannten Parameter die bevorzugten Untermenüs angezeigt sowie optional eine Auswahl, auf das "Standardmenü" zurückzugreifen, um bei Bedarf jederzeit auf die volle Funktionalität zugreifen zu können.

Die erfindungsgemässe Insulinpumpe kann - wie bereits erwähnt - neben einem Grundgerät auch eine Fernbedienung zum Steuern des Grundgeräts umfassen. Die Fernbedienung kann in ein Blutzuckermessgerät integriert sein. Das mindestens eine Bedienungselement zum Abfrufen der aufgrund des Präferenzprofils angebotenen Auswahlmöglichkeiten kann an der Fernbedienung und/oder am Grundgerät angeordnet. Ist das Bedienungselement am Grundgerät angeordnet, ist es aufgrund der neuartigen Benutzerschnittstelle nicht notwendig, die Fernbedienung dauernd mit sich zu führen, da die für den Benutzer wichtigen Funktionen auch direkt am Grundgerät komfortabel genutzt werden können.

Neben der Auswahl der anzuzeigenden Informationen und der anzubietenden Auswahl kann auch eine Reihenfolge von Auswahlmöglichkeiten eines Auswahlmenüs nach dem benutzerspezifischen Präferenzprofil ausgegeben werden, d. h. eine wahrscheinlichste Auswahl wird in Abhängigkeit der aktuellen Zeit und des Präferenzprofils prioritär dargestellt, und die weiteren Auswahlmöglichkeiten folgen in der Reihenfolge der ihnen zugeordneten Auswahlwahrscheinlichkeiten. "Prioritär" heisst in diesem Zusammenhang beispielsweise, dass die entsprechende Auswahlmöglichkeit am einfachsten und schnellsten auswählbar ist. Die prioritäre Auswahlmöglichkeit kann beispielsweise durch ein einfaches Drücken einer Bestätigungstaste ausgewählt werden, während zur Wahl einer weiteren Möglichkeit zunächst ein Zeiger darauf bewegt werden muss.

Mit Vorteil umfasst das Präferenzprofil mehrere Zeitebenen, die verschiedenen Zeiträumen entsprechen. Viele körperliche Zustände und Aktivitäten eines Benutzers wiederholen sich nämlich in der Regel in gewissen Zyklen mit unterschiedlichen Zykluszeiten. So sind beispielsweise im Rahmen eines erfindungsgemässen Verfahrens zur Steuerung der Benutzerschnittstelle folgende Ebenen denkbar:

| | | |
|---|---|---|
| infradian | jährlich | Feiertage |
| | halbjährlich | Therapieänderung nach Arztbesuch |
| | vierteljährlich | Therapieänderung nach Arztbesuch |
| | | Jahreszeiten |
| | wöchentlich | Arbeits-/Freitage |
| | | sportliche Betätigung |
| circadian | täglich | Schlaf-/Wachrhythmus |
| | | temporäre Basalraten |
| ultradian | ultradianer Rhythmus (6h) | Tagesanfang - Mittag - Abend - Nacht |
| | Mahlzeiten | pre-postprandiale Boluseingabe |

Weitere benutzerspezifische Gewohnheiten können auf jeder der Ebenen zusätzlich erfasst und berücksichtigt werden. Neben periodischen Angaben können auch Einzelereignisse vorgängig in das Präferenzprofil integriert werden, z. B. Ferien, Krankenhausaufenthalte, unregelmässige sportliche Aktivitäten etc.

Das Präferenzprofil kann gerätespezifische und systemspezifische Angaben umfassen. Dabei sind die gerätespezifischen Angaben spezifisch auf die Insulinpumpe und die systemspezifischen Angaben allgemein auf die für den Benutzer zugeschnittene Therapie bezogen. Bei den gerätespezifischen Angaben kann es sich beispielsweise um Kalibrationsparameter oder Messintervalle handeln, bei systemspezifischen Angaben beispielsweise um Glukosewerte, z. B. den Nüchtern-Glukose-Spiegel, Basalraten, typische Bolusmengen des Benutzers etc. Zumindest die systemspezifischen Angaben können über eine Schnittstelle zu anderen Geräten übermittelt und/oder von anderen Geräten empfangen werden. Somit wird ermöglicht, dass mehrere Geräte, die im Rahmen einer Therapie für unterschiedliche Zwecke eingesetzt werden, mit denselben systemspezifischen Angaben versorgt werden können, während die gerätespezifischen Informationen je nach Gerät unterschiedlich sind. Der Austausch der systemspezifischen Angaben kann automatisch erfolgen, beispielsweise über eine drahtlose Verbindung. Im Rahmen der Erfindung ist es bevorzugt möglich, das aktuell gespeicherte Präferenzprofil zu exportieren, um dieses zu sichern oder an ein anderes Gerät, beispielsweise eine Ersatz-Insulinpumpe, zu übermitteln.

Mit Vorteil umfasst das Präferenzprofil Angaben über mindestens einen meistbenutzten Befehl, wobei diese Angaben regelmässig aktualisiert werden. Dieser mindestens eine meistbenutzte Befehl ist über eine Sonderfunktion durch die Bedienungselemente direkt abrufbar. Diesem Befehl kann beispielsweise eine eigene Taste oder ein fester Platz in jedem Auswahlmenü zugewiesen sein. Der meistbenutzte Befehl kann in Abhängigkeit der aktuellen Zeit und gegebenenfalls von weiteren Angaben (z. B. über den aktuellen Zustand oder die aktuelle Aktivität des Benutzers) geändert werden, oder es handelt sich zeitunabhängig immer um denselben Befehl, wobei eine Änderung nur dann vorgenommen wird, wenn ein anderer Befehl über das Ganze gesehen häufiger benutzt wird.

Bevorzugt ist im Speicher eine Datenbank mit ortsspezifischen, für eine Insulintherapie relevanten Eigenheiten abgelegt, und das Präferenzprofil wird in Abhängigkeit eines aktuellen Standorts des Benutzers basierend auf der Datenbank angepasst. Die Datenbank kann beispielsweise Informationen über landesspezifische Essens- oder Ruhezeiten und Essgewohnheiten umfassen (beispielsweise über das späte Nachtessen oder eine mittägliche Ruhezeit in südlichen Ländern). Die landes-, bzw. standortspezifischen Informationen können auch mit Informationen zu Gewohnheiten des Benutzers verknüpft sein: Besitzt der Benutzer beispielsweise ein Feriendomizil, so wird er dort einen etwas anderen Lebensrhythmus haben als am Arbeits-, bzw. Wohnort. Stellt die Insulinpumpe fest, dass sich der Benutzer an diesem Feriendomizil befindet, bzw. wird ihr diese Information übermittelt oder eingegeben, berücksichtigt sie ein entsprechend angepasstes Präferenzprofil.

Entsprechend besitzt die Insulinpumpe bevorzugt Mittel zum Feststellen eines Standorts, insbesondere einen Empfänger für ein Navigationssystem oder eine Schnittstelle zu einem solchen Empfänger. Dadurch wird sichergestellt, dass die Steuerung der Insulinpumpe stets über den Aufenthaltsort des Benutzers informiert ist und die landes- bzw. standortspezifischen Informationen für die benutzerspezifische Steuerung der Benutzerschnittstelle berücksichtigen kann.

Alternativ wird der Standort vom Benutzer selbst angegeben, z. B. über die Bedienungselemente der Insulinpumpe.

Mit Vorteil ist die Insulinpumpe selbstlernend ausgebildet, wobei die Steuerung das benutzerspezifische Präferenzprofil aufgrund einer Analyse von Bedienungseingaben des Benutzers laufend aktualisiert. Die Steuerung korreliert beispielsweise die vom Benutzer erhaltenen Eingaben mit der jeweiligen Zeit und dem jeweiligen Datum, sowie gegebenenfalls mit weiteren zur Verfügung stehenden Daten. Durch eine statistische Analyse kann aus den verknüpften Daten in einem weiteren Schritt das Präferenzprofil gewonnen werden. Das Profil kann fortlaufend oder in gewissen Zeitabständen (z. B. täglich oder wöchentlich) aktualisiert werden. Bei der Aktualisierung werden kürzliche Eingaben bevorzugt stärker gewichtet als ältere Eingaben, wobei die Gewichtung je nach Zeitskala der Analyse unterschiedlich gewählt werden kann. Auch wenn das Präferenzprofil auf der Nutzung der Funktionen durch den Benutzer basiert, kann gewissen (z. B. sicherheitsrelevanten) Funktionen fest eine vorgegebene Gewichtung zugeordnet werden, so dass sie stets einfach aufrufbar sind.

Alternativ oder ergänzend wird das benutzerspezifische Präferenzprofil aufgrund einer Analyse von Bedienungseingaben des Benutzers während eines oder mehrerer Lernintervalle erstellt. So kann zum Beispiel ein Lernintervall von einigen Wochen nach Beginn der Benutzung der Insulinpumpe vorgesehen werden. Weitere Lernintervalle können folgen, wenn sich am Lebensrhythmus des Benutzers etwas grundlegend geändert hat.

Alternativ oder um ein erstes, vorläufiges Präferenzprofil zu gewinnen, kann bei der Einrichtung der Insulinpumpe bzw. im Rahmen einer Schulung eine Benutzeranalyse und/oder eine Benutzerbefragung durchgeführt werden. Die Informationen werden anschliessend über die Bedienungselemente der Insulinpumpe oder - bevorzugt - über ein externes, mit der Insulinpumpe verbundenes Gerät eingegeben. Das erfindungsgemässe Infusionssystem kann eine Benutzeroberfläche auf einem Gerät (der Insulinpumpe, einer Fernbedienung oder auf einem weiteren Gerät wie einem PC oder einem PDA) bereitstellen, mit welcher das Präferenzprofil benutzergesteuert angepasst werden kann und/oder welches die Verwaltung einer Mehrzahl von Präferenzprofilen ermöglicht.

Alternativ oder zusätzlich zur Auswertung der Benutzereingaben kann die Steuerung beim Erstellen bzw. Aktualisieren des benutzerspezifischen Präferenzprofils Daten eines Sensors zur Überwachung des Benutzers berücksichtigen. Geeignete Daten umfassen physiologische Daten wie Puls, Blutdruck, Körpertemperatur oder Blutzuckerspiegel, Informationen über Aktivitäten des Benutzers wie Bewegung, Beschleunigung, Aussentemperatur etc. oder Informationen über den Aufenthaltsort des Benutzers (siehe oben). Solche Daten können auch beim Darstellen und Anbieten der Auswahlmöglichkeiten mitberücksichtigt werden.

Die erfindungsgemässe Insulinpumpe kann auch eine Schnittstelle zu einem digitalen Zeitplaner umfassen, wobei dieser Zeitplaner auf einem PDA, einem Mobiltelefon, einem Notebook, einem Desktop-PC oder auf einer anderen Plattform realisiert sein kann. In solchen Zeitplanern sind üblicherweise für die Insulintherapie relevante Informationen abgelegt, beispielsweise Ferientermine oder Freitage und Angaben über Aktivitäten wie z. B. Sport. Werden diese Informationen für die Insulinpumpe verfügbar gemacht, kann bei der Steuerung der Benutzerschnittstelle noch spezifischer auf die Bedürfnisse des Benutzers eingegangen werden.

Bevorzugt ist die Steuerung derart ausgebildet und programmiert, dass bei einer Auswahl des Benutzers, welche von dem benutzerspezifischen Präferenzprofil abweicht, vor der Ausführung der Auswahl eine Sicherheitsabfrage (z. B. die Ausgabe "Sind Sie sicher?" mit der Aufforderung, die Auswahl noch einmal zu bestätigen) ausgegeben wird. Dadurch lässt sich auf einfache Weise die Sicherheit des erfindungsgemässen Infusionssystems erhöhen. Die Sicherheitsabfrage kann auf sicherheitsrelevante Eingaben beschränkt sein, z. B. auf die Ausschützung eines Bolus, die Änderung der Basalrate etc., während weitere Eingaben (z. B. die Auswahl einer Darstellungsart oder die reine Ausgabe von Information) ohne Rückfrage entgegengenommen und ausgeführt werden.

Mit Vorteil umfasst die Insulinpumpe einen Empfänger für ein Zeitsignal oder eine Schnittstelle zu einem solchen Empfänger. Entsprechende Zeitsignale sind in vielen Gegenden verfügbar, beispielsweise das Zeitsignal DCF77, das in West- und Mitteleuropa empfangen werden kann, oder Zeitsignale eines Mobilfunknetzes. Dies stellt sicher, dass die Insulinpumpe stets über die aktuelle Ortszeit verfügt und die entsprechende korrekte Basalrate injiziert. Wechsel der Zeitzone können zudem automatisch erkannt und bei der Auswertung des Präferenzprofils berücksichtigt werden.

Weiter umfasst die Insulinpumpe bevorzugt eine drahtlose Kommunikationsschnittstelle. Diese ermöglicht eine einfache Steuerung der Pumpe, beispielsweise durch eine Fernsteuerung, eine einfache Aktualisierung der Pumpenparameter und des Präferenzprofils, sowie ein einfaches Auslesen der in der Pumpe gespeicherten Informationen.

Ein Auslesen des aktuell gespeicherten Präferenzprofils ist deshalb sinnvoll, weil das Präferenzprofil charakteristische Informationen über den Umgang des Benutzers mit der Therapie liefert. Der HCP (Healthcare Provider) des Benutzers kann basierend auf dem ausgelesenen Profil Hilfestellung bei der Optimierung der Therapie bzw. für den Umgang des Benutzers mit der Therapie geben.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Blockdarstellung einer erfindungsgemässen Insulinpumpe;
- Fig. 2A, B: beispielhafte Anzeigen auf dem Display der erfindungsgemässen Insulinpumpe; und
- Fig. 3A-C: Histogramme über die Nutzung von verschiedenen Funktionen der Insulinpumpe im Tagesverlauf.

Grundsätzlich sind in den Figuren gleiche Teile mit gleichen Bezugszeichen versehen.

### Wege zur Ausführung der Erfindung

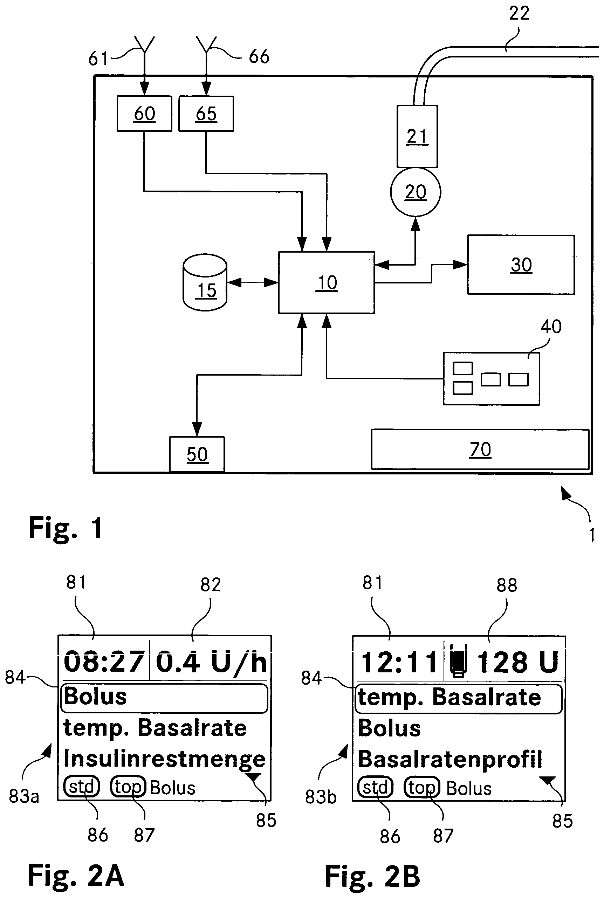

Die Figur 1 zeigt eine schematische Blockdarstellung einer erfindungsgemässen Insulinpumpe. Die Insulinpumpe 1 umfasst eine zentrale Steuerung 10, welche durch einen Mikroprozessor realisiert ist. Diese Steuerung 10 kann Daten in einem Speicher 15 ablegen, bzw. aus diesem abrufen. Die zentrale Steuerung 10 steuert die Pumpe 20, die einen Motor und ein Getriebe umfasst und die Insulin je nach Bedarf des Benutzers aus einer Ampulle 21 in einen Infusionsschlauch 22 eines Infusionssets fördert. Die Insulinpumpe 1 weist im Weiteren ein von der Aussenseite ablesbares Display 30 sowie Bedientasten 40 auf, die jeweils mit der Steuerung 10 zur Ausgabe bzw. Eingabe von Informationen verbunden sind. Im Weiteren weist die dargestellte Insulinpumpe 1 ein ebenfalls mit der zentralen Steuerung 10 verbundenes Kommunikationsmodul 50 auf mit drahtlosen und drahtgebundenen Schnittstellen zur Kommunikation mit weiteren Geräten, insbesondere mit einer Fernbedienung (die als Teil eines Blutzuckermessgeräts ausgebildet sein kann), mit einem digitalen Zeitplaner (z. B. einem PDA), einem Personal Computer und einer weiteren Insulinpumpe.

Die Insulinpumpe 1 umfasst zudem einen Empfänger 60 für ein Navigationssystem mit einer entsprechenden Antenne 61 und einen Empfänger 65 für ein Zeitsignal mit einer entsprechenden Antenne 66, wobei die Empfänger 60, 65 wiederum mit der zentralen Steuerung 10 verbunden sind. Die einzelnen Komponenten der Insulinpumpe 1 werden von einer integrierten Batterie 70 mit Strom versorgt.

Die Figuren 2A, 2B zeigen zwei beispielhafte Anzeigen auf dem Display der erfindungsgemässen Insulinpumpe. Die in der Figur 2A dargestellte erste Anzeige umfasst Informationen über die aktuelle Uhrzeit 81, die aktuelle Insulinfördermenge 82 in Einheiten (I. E.) pro Stunde (units per hour) sowie eine Menüauswahl 83a mit drei Menüpunkten
a) Bolus,
b) temp. Basalrate,
c) Insulinrestmenge,
einem Zeiger 84, der auf den ersten angezeigten Menüpunkt ("Bolus") gerichtet ist und einer pfeilartigen Anzeige 85, die darauf hinweist, dass weitere Menüpunkte vorhanden sind.

Welche Information dargestellt wird (Uhrzeit 81, Insulinfördermenge 82), welche Auswahloptionen (Bolus, temp. Basalrate, Insulinrestmenge) zur Verfügung gestellt und in welcher Reihenfolge sie angezeigt werden wird - wie weiter unten näher beschrieben - durch das Präferenzprofil des Benutzers bestimmt. Der Zeiger 84 ist jeweils auf den ersten dargestellten Menüpunkt gerichtet, so dass dieser durch einen einfachen Tastendruck ("Bestätigen") ausgewählt werden kann. Die Anzeige 86 ("std") weist darauf hin, dass durch Druck auf eine entsprechend gekennzeichnete Taste in eine Standard-Menüstruktur der Insulinpumpe gewechselt werden kann, die zeitlich unveränderlich ist und über welche sämtliche verfügbaren Funktionen abgerufen werden können. Die Anzeige 87 ("top") weist darauf hin, dass durch Druck auf eine entsprechend gekennzeichnete Taste der vom Benutzer meistbenutzte Befehl (hier "Bolus") auswählbar ist.

Die Figur 2B stellt eine zweite Anzeige zu einem anderen Zeitpunkt dar. Diese zeigt wiederum die Uhrzeit 81, nun aber das in der Ampulle verbliebene Insulin (Insulinrestmenge 88) in Einheiten (units). Auch die zweite Anzeige umfasst im Weiteren eine Menüauswahl 83b, die nun aber teilweise andere Menüpunkte und eine andere Reihenfolge aufweist:
a) temp. Basalrate,
b) Bolus,
c) Basalratenprofil,
wiederum ist der Zeiger 84 auf den ersten angezeigten Menüpunkt ("temp. Basalrate") gerichtet, und wiederum ist eine pfeilartige Anzeige 85 vorhanden, die darauf hinweist, dass weitere Menüpunkte vorhanden sind sowie die Anzeigen 86, 87, die auf die Verfügbarkeit einer Standard-Menüstruktur sowie eines Direktaufrufs für die meistbenutzte Funktion hinweisen.

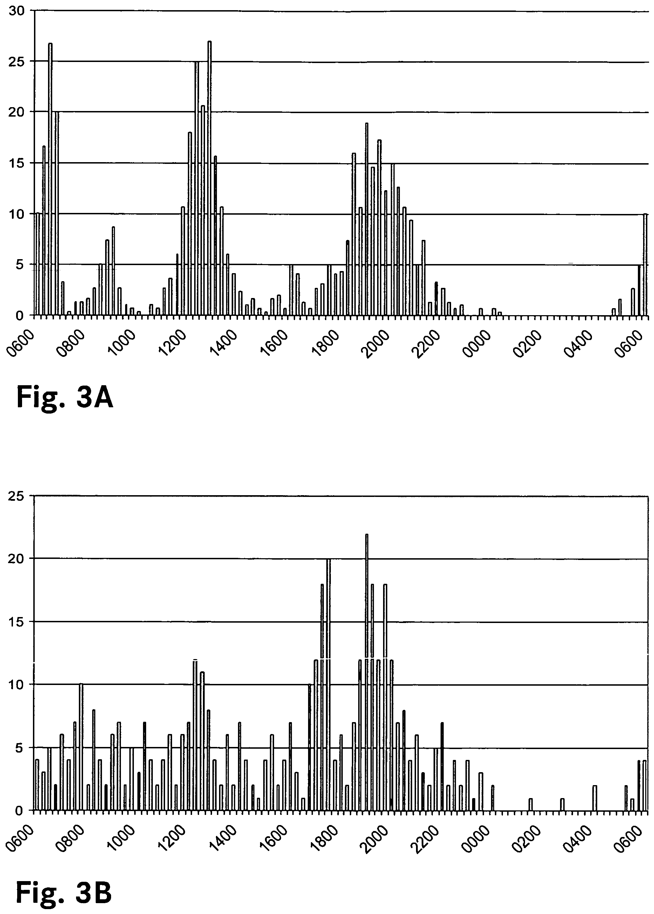

Die Figuren 3A-3C zeigen Histogramme über die Nutzung von drei verschiedenen Funktionen der Insulinpumpe im Tagesverlauf (von 6 Uhr morgens bis 6 Uhr morgens des nächsten Tages), wobei die Aktionen des Benutzers über einen längeren Zeitraum (z. B. 3 Monate) erfasst wurden und in diesen Diagrammen dargestellt sind.

Die Figur 3A zeigt die Nutzungsverteilung einer Funktion, welche klare Maxima aufweist, nämlich um ca. 7 Uhr, um ca. 9 Uhr, zwischen 12.30 und 13.30 Uhr und zwischen 19 und 21 Uhr. Es handelt sich somit um eine Funktion (z. B. die Verabreichung eines Bolus), welche der Benutzer zu gewissen Zeiten regelmässig, ausserhalb dieser Zeiten aber nur sehr selten aufruft.

Die Figur 3B zeigt die Nutzungsverteilung einer weiteren Funktion, welche gewisse Maxima und Minima aufweist, insbesondere in der späteren Mittagszeit, am Vorabend und nach 20 Uhr. Im Gegensatz zur Verteilung der ersten Funktion gemäss Figur 3A sind die Maxima aber nicht so deutlich, und zumindest während des Tages wird diese Funktion regelmässig auch zwischen den erwähnten Maxima aufgerufen. Eine entsprechende Verteilung ergibt sich beispielsweise bei einer Funktion wie "Temporäre Basalratenänderung".

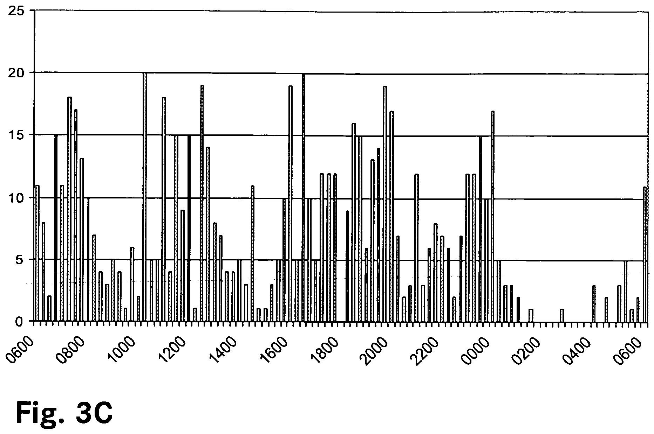

Die Figur 3C zeigt ein Histogramm einer weiteren Funktion. Hier ist - mit Ausnahme der geringen Aufrufhäufigkeit in den Nachtstunden - keine klare Tendenz erkennbar.

Entsprechende Nutzungsdaten werden von der erfindungsgemässen Insulinpumpe für sämtliche an der Insulinpumpe vorhandenen Funktionen, inklusive der eingestellten Darstellung auf dem Display, automatisch erhoben und neben der Zeitinformation mit weiteren Informationen verknüpft (z. B. Wochentag, Jahreszeit, Aufenthaltsort des Benutzers, Puls, allenfalls verfügbare Informationen aus dem Zeitplaner etc.). Dies erlaubt eine nachträgliche Aufschlüsselung der Nutzungsfrequenz nach den erwähnten Kriterien.

Typische Anweisungen einer Insulinpumpe, deren Nutzungshäufigkeit erfasst werden kann, sind die Folgenden:
a) Basalratenprofil wählen;
b) Bolus-Speicher anzeigen;
c) Bolus auslösen (Standard, verzögert);
d) temporäre Basalratenänderung (Erhöhung/Senkung) ;
e) Starten und Anhalten der Insulinabgabe;
f) Bolus-Schrittweite eingeben;
g) Uhrzeit / Zeitzone einstellen;
h) Basalratenprofil programmieren.

Anzeigefunktionen, deren Häufigkeit ebenfalls erfasst wird, umfassen:
a) aktuelle Basalrate (in Abhängigkeit des Profils) in U/h;
b) Insulinrestmenge in U;
c) Uhrzeit / Datum;
d) Fehlermeldungen / Alarme;
e) Batteriestand;
f) Einstellungen, Menüs, Untermenüs;
g) Betriebszustand (STOP / RUN);
h) frühere Boli (Uhrzeit, Datum, Menge);
i) Tagesmengen.

Aus den Analysen ergibt sich das benutzerspezifische Präferenzprofil. Die folgende Tabelle zeigt einen vereinfachten Ausschnitt aus einem beispielhaften Präferenzprofil, wobei der Einfachheit halber nur eine Aufschlüsselung nach Zeit (halbstündige Abschnitte) und nach Arbeitstag/Freitag vorgenommen wurde, und wobei nur fünf Anweisungen/Anzeigefunktionen dargestellt sind. Die angegebenen Zahlen stellen den jeweiligen Präferenzwert der Funktion im gegebenen Zeitintervall und am gegebenen Wochentag (in beliebigen Einheiten) dar, der jeweils höchste Wert ist umrahmt:

| | | Opt1 | Opt2 | Opt3 | Opt4 | Opt5 |
|---|---|---|---|---|---|---|
| 0600 | Mo-Fr | 24 | 4 | 11 | 6 | 8 |
| | Sa/Su | 8 | 4 | 8 | 4 | 8 |
| 0630 | Mo-Fr | 32 | 3 | 13 | 4 | 7 |
| | Sa/Su | 9 | 2 | 9 | 3 | 7 |
| 0700 | Mo-Fr | 25 | 5 | 22 | 5 | 9 |
| | Sa/Su | 10 | 5 | 8 | 4 | 9 |
| 0730 | Mo-Fr | 21 | 7 | 17 | 7 | 12 |
| | Sa/Su | 12 | 4 | 11 | 5 | 12 |
| 0800 | Mo-Fr | 14 | 24 | 12 | 3 | 11 |
| | Sa/Su | 19 | 14 | 12 | 1 | 11 |
| 0830 | Mo-Fr | 10 | 11 | 8 | 10 | 9 |
| | Sa/Su | 22 | 11 | 14 | 3 | 9 |
| 0900 | Mo-Fr | 11 | 7 | 13 | 8 | 8 |
| | Sa/Su | 24 | 12 | 15 | 6 | 8 |
| 0930 | Mo-Fr | 8 | 5 | 12 | 11 | 3 |
| | Sa/Su | 18 | 14 | 10 | 7 | 3 |
| 1000 | Mo-Fr | 7 | 11 | 13 | 14 | 2 |
| | Sa/Su | 14 | 16 | 8 | 10 | 2 |
| 1030 | Mo-Fr | 7 | 8 | 13 | 12 | 1 |
| | Sa/Su | 5 12 | 14 | 11 | 14 | 1 |
| 1100 | Mo-Fr | | 14 | 11 | 8 | 0 |
| | Sa/Su | 8 | 14 | 14 | 15 | 0 |
| 1130 | Mo-Fr | 18 | 15 | 12 | 4 | 0 |
| | Sa/Su | 14 | 12 | 10 | 11 | 0 |
| 1200 | Mo-Fr | 26 | 22 | 13 | 6 | 1 |
| | Sa/Su | 12 | 14 | 7 | 9 | 1 |
| 1230 | Mo-Fr | 29 | 18 | 8 | 5 | 0 |
| | Sa/Su | 13 | 11 | 5 | 6 | 0 |
| 1300 | Mo-Fr | 25 | 10 | 11 | 5 | 2 |
| | Sa/Su | 10 | 10 | 9 | 4 | 2 |
| 1330 | Mo-Fr | 21 | 12 | 9 | 4 | 0 |
| | Sa/Su | 7 | 14 | 9 | 2 | 0 |
| 1400 | Mo-Fr | 8 | 14 | 13 | 5 | 1 |
| | Sa/Su | 8 | 15 | 14 | 7 | 1 |
| 1430 | Mo-Fr | ... | ... | ... | ... | ... |

Aus der Tabelle ist ersichtlich, dass die Nutzungshäufigkeiten zwischen Arbeits- und Freitag teilweise recht unterschiedlich sind. Die Insulinpumpe ist nun beispielsweise so gesteuert, dass sich (wie in den Figuren 2A, 2B dargestellt) an einem Samstag um 08.27 Uhr die Reihenfolge Bolus (Opt 1) / temporäre Basalrate (Opt2) / Darstellung Insulinrestmenge (Opt 3) ergibt, während sich am selben Tag um 12.11 Uhr die Reihenfolge temporäre Basalrate / Bolus / Basalratenprofil (Opt 4) ergibt. Entsprechend werden die angebotenen Auswahlmöglichkeiten und deren Reihenfolge angepasst.

In der realen Ausführung wird nicht nur nach Tageszeit und Arbeitstag/Wochenende unterschieden, sondern es fliessen - wie oben erwähnt -weitere Faktoren ein. Diesen kann Rechnung getragen werden, indem die Tabelle entsprechend zusätzliche Zeilen für weitere Parameterkombinationen aufweist oder indem die Präferenzwerte zu einem gegebenen Zeitpunkt in an sich bekannter Weise aus Teilpräferenzwerten errechnet werden. Diese Teilpräferenzwerte können beispielsweise einer ultradianen, einer circadianen und einer infradianen Teilpräferenz entsprechen, die für den gegebenen Zeitpunkt summiert werden.

Liegt eine Information über besondere Bedingungen oder Aktivitäten des Benutzers vor (beispielsweise eine Information aus dem Zeitplaner, wonach der Benutzer kurz nach dem aktuellen Zeitpunkt eine sportliche Aktivität eingeplant hat), kann diesen Umständen im Rahmen des erfindungsgemässen Verfahrens Rechnung getragen werden; beispielsweise werden die Präferenzwerte in einem gewissen Zeitraum vor sowie während der sportlichen Betätigung einem speziellen "Sportprofil" entnommen. Dieses wird - wie das übliche Präferenzprofil - stets dann aufgrund der erfolgten Benutzereingaben aktualisiert, wenn es aktiv ist. Ähnliche Spezialprofile können beim Vorliegen weiterer Situationen aufgerufen werden ("Reise", "spätes Essen", "Urlaub" etc.). Bei einem Schichtarbeiter ist es auch beispielsweise denkbar, dass ein "Tagschichtprofil" und ein "Nachtschichtprofil" vorhanden sind. Die Auswahl eines anderen Profils kann automatisiert erfolgen, aber auch manueii durch den Benutzer. Eine Mehrzahl von Profilen kann definiert werden, jeweils eines davon wird jeweils als aktuell herangezogenes Präferenzprofil ausgewählt. Die einzelnen Profile können getrennt voneinander erzeugt, eingelesen, modifiziert oder exportiert werden.

Wird mit Hilfe des Empfängers für das Zeitsignal und / oder des Empfängers für das Navigationssystem festgestellt, dass die Zeitzone gewechselt wird, erfolgt automatisch eine Umstellung der internen Zeit der Insulinpumpe und entsprechend eine Anpassung der auszuschüttenden (basalen) Insulinmenge und der darzustellenden Informationen und Benutzerpräferenzen.

Bestätigt der Benutzer eine Auswahl, die nicht seinem Präferenzprofil entspricht (somit entsprechend einen vorgegebenen Minimal-Präferenzwert unterschreitet) und die relevant für die Gesundheit des Benutzers ist (also beispielsweise die sofortige oder zukünftige Abgabe von Insulin beeinflusst), erfolgt vor der Ausführung der ausgewählten Funktion eine zusätzliche Sicherheitsabfrage, die vom Benutzer zu quittieren ist.

Vor der ersten Inbetriebnahme der Insulinpumpe wird ein Anfangs-Präferenzprofil vorgegeben. Dieses kann beispielsweise aufgrund einer Befragung des Benutzers oder aufgrund von Informationen, die über den Benutzer bereits vorliegen, bereits benutzerspezifisch eingerichtet werden. Hat der Benutzer bereits vorher eine Insulinpumpe mit Präferenzprofil benutzt, kann das Präferenzprofil der früheren Pumpe übernommen werden. Alternativ wird ein vorgegebenes allgemeines Anfangsprofil in der Insulinpumpe gespeichert. Aufgrund der Selbstlernfunktion der Insulinpumpe wird sich das benutzerspezifische Präferenzprofil im Rahmen der täglichen Nutzung von selbst einstellen.

Das Anfangs-Präferenzprofil kann extern erstellt und über die Schnittstellen des Kommunikationsmoduls 50 eingelesen werden. Ähnlicherweise wird eine Analyse, manuelle Anpassung oder Bearbeitung des Präferenzprofils mit Vorteil extern (z. B. auf einem PC) vorgenommen und das geänderte Profil anschliessend in die Pumpe eingelesen. Die Export- und Importmöglichkeiten für das Profil schaffen auch eine einfache Möglichkeit zum Anlegen von Sicherheitskopien des Profils und/oder der zugrundeliegenden Informationen.

Die Erfindung ist nicht auf das dargestellte Ausführungsbeispiel beschränkt, insbesondere kann die Insulinpumpe anders ausgebildet sein und zusätzliche Bauteile aufweisen bzw. nicht über Elemente wie einen Empfänger für ein Navigationssystem oder für ein Zeitsignal verfügen. Auch die Informationsanzeige und Menüführung der Insulinpumpe wird in der Regel anders gestaltet sein, z. B. unter Einsatz einer Reihe von grafischen Elementen. Steht nur eine kleinflächige Anzeige zur Verfügung, kann jeweils auch nur eine Auswahlmöglichkeit angezeigt werden, wobei mit Hilfe eines Bedienungselements zu weiteren Auswahlmöglichkeiten gewechselt ("gescrollt") werden kann. In diesem Fall bestimmt das Präferenzprofil die Reihenfolge, in welcher die Auswahlmöglichkeiten dargestellt werden.

Die Aktualisierung des Präferenzprofils und die Ermittlung der präferierten Funktionen und Anzeigen kann auf vielerlei Weise erfolgen, entsprechende Methoden sind bekannt. Als Variante zum oben beschriebenen Verfahren kann das Präferenzprofil während eines vorgegebenen Lernintervalls auf der Basis der vom Benutzer vorgenommenen Bedien- und Auswahloperationen erzeugt werden. Nach Ende des Lernintervalls können die Operationen des Benutzers weiter erfasst und zum Präferenzprofil in Beziehung gesetzt werden. Ergibt die Analyse, dass vom Präferenzpröfil häufig bzw. statistisch signifikant abgewichen wird, kann dies dem Benutzer mitgeteilt und die Erzeugung eines neuen Präferenzprofils vorgeschlagen werden.

Bei der Erzeugung des Präferenzprofils im Lernintervall oder bei dessen laufender Aktualisierung können bestimmte nicht-charakteristische Zeiten blockiert bzw. von der statistischen Analyse zur Erzeugung bzw. Modifikation des Präferenzprofils ausgenommen werden.

Zusammenfassend ist festzustellen, dass durch die Erfindung eine Insulinpumpe und ein Verfahren zur Steuerung einer Benutzerschnittstelle einer solchen Insulinpumpe geschaffen werden, welche einen hohen Funktionsumfang und eine einfache Bedienung auch auf miniaturisierten Pumpen ermöglichen.

## Patentansprüche

1. Insulinpumpe (1), welche selbstlernend ausgebildet ist, mit einer Pumpeinrichtung (20), einer Steuerung (10) für die Pumpeinrichtung (20), einem Speicher (15), mindestens einem Bedienungselement (40) für den Benutzer der Insulinpumpe (1) zur Bedienung der Insulinpumpe (1) und einer Anzeigeeinrichtung (30), wobei die Steuerung (10) derart ausgebildet und programmiert ist, dass ein benutzerspezifisches, im Speicher (15) abgelegtes Präferenzprofil aufgrund einer Analyse von Bedienungseingaben des Benutzers laufend aktualisiert wird, **dadurch gekennzeichnet, dass** die Analyse von Bedienungseingaben das Korrelieren mit der jeweiligen Zeit und dem jeweiligen Datum sowie eine statistische Analyse zur Gewinnung des aktuellen benutzerspezifischen Präferenzprofils umfasst, wobei dem Benutzer der Insulinpumpe (1) auf der Anzeigeeinrichtung (30) eine Menüauswahl mit Menüpunkten in Abhängigkeit des aktuellen benutzerspezifischen Präferenzprofils und einer aktuellen Zeit angeboten werden, wobei die Reihenfolge der Menüpunkte der Menüauswahl gemäss dem aktuellen Präferenzprofil und der aktuellen Zeit ausgegeben wird, wobei
der Menüpunkt mit der wahrscheinlichsten Auswahl in Abhängigkeit der aktuellen Zeit und des Präferenzprofils prioritär dargestellt wird und die weiteren Menüpunkte in der Reihenfolge der ihnen zugeordneten Auswahlwahrscheinlichkeiteri folgen, wobei prioritär bedeutet, dass der betreffende Menüpunkt durch Drücken einer Bestätigungstaste ausgewählt werden kann, während zur Wahl eines weiteren Menüpunkts zunächst ein Zeiger darauf bewegt werden muss.

2. Insulinpumpe (1) nach Anspruch 1, **dadurch gekennzeichnet, dass** das benutzerspezifische Präferenzprofil aufgrund einer Analyse von Bedienungseingaben des Benutzers während eines oder mehrerer Lernintervalle erstellt wird.

3. Insulinpumpe (1) nach Anspruch 1 oder 2, **dadurch gekennzeichnet, dass** ein Sensor zur Überwachung des Benutzers vorhanden ist und dass die Steuerung (10) Daten des Sensors beim Erstellen bzw. Aktualisieren des benutzerspezifischen Präferenzprofils und/oder beim Darstellen und Anbieten der Menüauswahl mit Menüpunkten berücksichtigt

4. Insulinpumpe (1) nach einem der Ansprüche 1 bis 3, **dadurch gekennzeichnet, dass** das Präferenzprofil mehrere Zeitebenen umfasst, die verschiedenen Zeiträumen, insbesondere infradian, circadian und ultradian, entsprechen.

5. Insulinpumpe (1) nach einem der Ansprüche 1 bis 4, **dadurch gekennzeichnet, dass** das Präferenzprofil gerätespezifische und systemspezifische Angaben umfasst, wobei die gerätespezifischen Angaben spezifisch auf die Insulinpumpe (1) und die systemspezifischen Angaben allgemein auf die für den Benutzer zugeschnittene Therapie bezogen sind und wobei die systemspezifischen Angaben über eine Schnittstelle zu anderen Geräten übermittelt und/oder von anderen Geräten empfangen werden können.

6. Insulinpumpe (1) nach einem der Ansprüche 1 bis 5, **gekennzeichnet durch** eine drahtlose Kommunikationsschnittstelle (50).

7. Verfahren zur Steuerung einer Benutzerschnittstelle einer selbstlernend ausgebildeten Insulinpumpe (1), wobei ein benutzerspezifisches, in einem Speicher (15) abgelegtes Präferenzprofil aufgrund einer Analyse von Bedienungseingaben des Benutzers laufend aktualisiert wird, **dadurch gekennzeichnet, dass** die Analyse von Bedienungseingaben das Korrelieren mit der jeweiligen Zeit und dem jeweiligen Datum sowie eine statistische Analyse zur Gewinnung des aktuellen benutzerspezifischen Präferenzprofils umfasst, wobei auf einer Anzeigeeinrichtung (30) eine Menüauswahl mit Menüpunkten für den Benutzer der Insulinpumpe (1) in Abhängigkeit des aktuellen benutzerspezifischen Präferenzprofils und einer aktuellen Zeit angeboten werden, wobei die Reihenfolge der Menüpunkte der Menüauswahl gemäss dem aktuellen Präferenzprofil und der aktuellen Zeit ausgegeben wird, wobei der Menüpunkt mit der wahrscheinlichsten Auswahl in Abhängigkeit der aktuellen Zeit und des Präferenzprofils prioritär dargestellt wird und die weiteren Menüpunkte in der Reihenfolge der ihnen zugeordneten Auswahlwahrscheinlichkeiten folgen, wobei prioritär bedeutet, dass der betreffende Menüpunkt durch Drücken einer Bestätigungstaste ausgewählt werden kann, während zur Wahl eines weiteren Menüpunkts zunächst ein Zeiger darauf bewegt werden muss.

## Claims

1. An insulin pump (1) which is self-learning in configuration, having a pump device (20), a control (10) for the pump device (20), a memory (15), at least one operating element (40) for the user of the insulin pump (1) to operate the insulin pump (1), and a display device (30), wherein the control (10) is designed and programmed in a manner such that a user-specific preference profile stored in the memory (15) is continuously updated on the basis of an analysis of operating inputs by the user, **characterized in that** the analysis of the operating inputs comprises correlating with the respective time and with the respective date as well as a statistical analysis in order to obtain the updated user-specific preference profile, wherein a menu selection with menu items which are a function of the updated user-specific preference profile and a current time are presented to the user of the insulin pump (1) on the display device (30), wherein the menu items of the menu selection are displayed in an order which is in accordance with the updated preference profile and the current time, wherein
the menu item with the most probable selection as a function of the current time and of the preference profile is displayed in a prioritized manner and the further menu items follow in the order of the selection probabilities assigned thereto, wherein prioritized means that the relevant menu item can be selected by pressing a confirmation key, whereas in order to select a further menu item, a cursor must firstly be moved onto it.

2. The insulin pump (1) as claimed in claim 1, **characterized in that** the user-specific profile is generated on the basis of an analysis of operating inputs by the user during one or more learning intervals.

3. The insulin pump (1) as claimed in claim 1 or claim 2, **characterized in that** a sensor is provided for monitoring the user, and **in that** the control (10) takes data from the sensor into account when generating or updating the user-specific preference profile and/or when displaying and presenting the menu selection with menu items.

4. The insulin pump (1) as claimed in one of claims 1 to 3, **characterized in that** the preference profile comprises a plurality of time domains which correspond to different time intervals, in particular infradian, circadian and ultradian time intervals.

5. The insulin pump (1) as claimed in one of claims 1 to 4, **characterized in that** the preference profile comprises equipment-specific and system-specific information, wherein the equipment-specific information specifically relates to the insulin pump (1), and the system-specific information generally relates to the therapy tailored to the user, and wherein the system-specific information can be transmitted to other equipment and/or received from other equipment via an interface.

6. The insulin pump (1) as claimed in one of claims 1 to 5, **characterized by** a wireless communication interface (50) .

7. A method for controlling a user interface of an insulin pump (1) which is configured so as to be self-learning, wherein a user-specific preference profile stored in a memory (15) is continuously updated on the basis of an analysis of operating inputs by the user, **characterized in that** the analysis of the operating inputs comprises correlating with the respective time and with the respective date as well as a statistical analysis in order to obtain the updated user-specific preference profile, wherein a menu selection with menu items which are a function of the updated specific preference profile for the user of the insulin pump (1) and a current time are presented on a display device (30), wherein the menu items of the menu selection are displayed in an order which is in accordance with the updated preference profile and the current time, wherein the menu item with the most probable selection as a function of the current time and of the preference profile is displayed in a prioritized manner and the further menu items follow in the order of the selection probabilities assigned thereto, wherein prioritized means that the relevant menu item can be selected by pressing a confirmation key, whereas in order to select a further menu item, a cursor must firstly be moved onto it.

## Revendications

1. Pompe à insuline (1), laquelle est conçue pour l'auto-apprentissage, comprenant un dispositif de pompe (20), une commande (10) pour le dispositif de pompe (20), une mémoire (15), au moins un élément de manipulation (40) pour l'utilisateur de la pompe à insuline (1) pour utiliser la pompe à insuline (1) et un dispositif d'affichage (30), dans laquelle la commande (10) est conçue et programmée de façon à ce qu'un profil préférentiel spécifique à l'utilisateur mis dans la mémoire (15) soit actualisé en continu en se basant sur une analyse de saisies de manipulation de l'utilisateur, **caractérisée en ce que** l'analyse de saisies de manipulation comprend la corrélation avec le temps respectif et la date respective ainsi qu'une analyse statistique afin d'obtenir le profil préférentiel actuel spécifique à l'utilisateur, dans laquelle une sélection de menu avec des points de menu en fonction du profil préférentiel actuel spécifique à l'utilisateur et d'un temps actuel est proposée à l'utilisateur de la pompe à insuline (1) sur le dispositif d'affichage (30), dans laquelle la séquence des points de menu de la sélection de menu est fournie selon le profil préférentiel actuel et le temps actuel, dans laquelle
le point de menu avec la sélection la plus probable en fonction du temps actuel et du profil préférentiel est affiché de manière prioritaire et les autres points de menu dans la séquence suivent les probabilités de sélection qui leur sont attribuées, dans laquelle prioritaire signifie que le point de menu concerné peut être sélectionné en appuyant sur une touche de validation, tandis que pour sélectionner un autre point de menu, un pointeur doit être d'abord déplacé dessus.

2. Pompe à insuline (1) selon la revendication 1, **caractérisée en ce que** le profil préférentiel spécifique à l'utilisateur est créé en se basant sur une analyse de saisies d'utilisation de l'utilisateur pendant un ou plusieurs intervalle(s) d'apprentissage.

3. Pompe à insuline (1) selon la revendication 1 ou 2, **caractérisée en ce qu'**un capteur pour surveiller l'utilisateur est présent et que la commande (10) tient compte des données du capteur lors de la création, respectivement de l'actualisation du profil préférentiel spécifique à l'utilisateur et/ou lors de l'affichage et de la proposition de sélection de menu par les points de menu.

4. Pompe à insuline (1) selon l'une des revendications 1 à 3, **caractérisée en ce que** le profil préférentiel comprend plusieurs niveaux temporels qui correspondent aux différentes périodes, en particulier infradienne, circadienne et ultradienne.

5. Pompe à insuline (1) selon l'une des revendications 1 à 4, **caractérisée en ce que** le profil préférentiel comprend des indications spécifiques à l'appareil et spécifiques au système, dans laquelle les indications spécifiques à l'appareil se rapportent spécifiquement à la pompe à insuline (1) et les indications spécifiques au système se rapportent généralement au traitement conçu pour l'utilisateur et dans laquelle les indications spécifiques au système peuvent être transmises à d'autres appareils et/ou être reçues par d'autres appareils par le biais d'une interface.

6. Pompe à insuline (1) selon l'une des revendications 1 à 5, **caractérisée par** une interface de communication sans fil (50).

7. Procédé de commande d'une interface utilisateur d'une pompe à insuline (1) qui est conçue pour l'auto-apprentissage, dans lequel un profil préférentiel spécifique à l'utilisateur mis dans une mémoire (15) est actualisé en continu en se basant sur une analyse de saisies de manipulation de l'utilisateur, **caractérisé en ce que** l'analyse de saisies de manipulation comprend la corrélation avec le temps respectif et la date respective ainsi qu'une analyse statistique afin d'obtenir le profil préférentiel actuel spécifique à l'utilisateur, dans lequel une sélection de menu avec des points de menu pour l'utilisateur de la pompe à insuline (1) en fonction du profil préférentiel actuel spécifique à l'utilisateur et d'un temps actuel est proposée sur un dispositif d'affichage (30), dans lequel la séquence des points de menu de la sélection de menu est fournie selon le profil préférentiel actuel et le temps actuel, dans lequel
le point de menu avec la sélection la plus probable en fonction du temps actuel et du profil préférentiel est affiché de manière prioritaire et les autres points de menu dans la séquence suivent les probabilités de sélection qui leur sont attribuées, dans lequel prioritaire signifie que le point de menu concerné peut être sélectionné en appuyant sur une touche de validation, tandis que pour sélectionner un autre point de menu, un pointeur doit être d'abord déplacé dessus.
